Europäisches Patentamt

European Patent Office

Office européen des brevets

(11) Publication number: **0 475 425 A1**

# EUROPEAN PATENT APPLICATION

(21) Application number: **91115538.0**

(22) Date of filing: **13.09.91**

(51) Int. Cl.5: **A61M 35/00**, A45D 34/04,
B05C 17/00

(30) Priority: **14.09.90 JP 244266/90**

(43) Date of publication of application:
**18.03.92 Bulletin 92/12**

(84) Designated Contracting States:
**BE DE FR GB IT NL SE**

(71) Applicant: **TERUMO KABUSHIKI KAISHA**
**No. 44-1, Hatagaya 2-chome, Shibuya-ku**
**Tokyo 151(JP)**

(72) Inventor: **Hirose, Kazunori, c/o Terumo**
**Kabushiki Kaisha**
**1727-1, Tsuijiarai, Showa-cho**
**Nakakoma-gun, Yamanashi-ken(JP)**

(74) Representative: **Casalonga, Axel et al**
**BUREAU D.A. CASALONGA - JOSSE**
**Morassistrasse 8**
**W-8000 München 5(DE)**

(54) **Liquid applicator.**

(57) A liquid applicator having a container (2) which can be broken at its thin-walled portion (5), into a main body (3) and a cap (4). The main body (3) has a space filled with a liquid (8). A columnar liquid-applying member (6) is located in the container (3), partly located in the main body (3) and partly in the cap (4). The liquid-applying member (6) is connected to the main body (3) by a coil spring (9) arranged in the space. The liquid-applying member (6) is supplied with the liquid (8) from the space and is impregnated with the liquid (8). A hydrophobic film (7) covers the substantially entire circumferential surface of the liquid-applying member (6). The film contacts the inner surface of the main body (3), but can move in the axial direction thereof together with the liquid-appliying member (6). The film (7) prevents the liquid from being scattered around when the container (2) is broken at its thin-walled portion (5).

F I G. 1

The present invention relates to a liquid applicator for applying liquid, such as a liquid medicine, to a limited surface of a living body.

Liquid applicators have been developed and used in various fields. Prominent use of liquid applicators is found in medicine; applicators are used to apply liquid medicine to patients' affected portions.

Various types of liquid applicators have been developed for medical use, which are easy to manipulate and sufficient hygienic. Among these liquid applicators is a type which has a container which can be broken by hands and a liquid-applying member contained in the container impregnated with liquid medicine. Liquid applicators of this type are disclosed in Published Unexamined Japanese Patent Application No. 63-181776 and PCT Application PCT/JP88/01234 (i.e., Japanese Patent Application No. 62-315251). In use, the container is broken, thus exposing part of the liquid-applying member, and the exposed part of the member is brought into contact with the affected portion of a patient, whereby the liquid medicine is applied to the affected portion.

This type of a liquid applicator type has a draw back. When the container is broken, the resultant impact is transmitted to the liquid-applying member. Consequently the member vibrates, and the liquid medicine inevitably scatter around, wetting the fingers of the person who hold the applicator or the clothes that person wears.

The object of this invention is to provide a liquid applicator which is easy to manipulate and sufficiently hygienic and which does not scatter liquid around when opened to apply the liquid.

In a first aspect of the invention, there is provided a liquid applicator comprising:

a container comprising a main body, a detachable portion, and a connecting means connecting the container and the main body together;

space-defining means defining a space in the main body;

an amount of liquid contained in said space;

a columnar liquid-applying member made of liquid-holding material, located within the container and secured to the main body, supplied with the liquid from the space, and having a distal end from which to apply the liquid; and

a hydrophobic film surrounding a circumferential surface of at least that portion of the liquid-applying member which protrudes from the main body.

In a second aspect of the present invention, there is provided a liquid applicator comprising:

a container comprising a main body, a detachable portion, and a connecting means connecting the container and the main body together;

space-defining means defining a space in the main body;

an amount of liquid contained in said space;

a columnar liquid-applying member made of liquid-holding material, located within the container and extending between the main body and the detachable portion, supplied with the liquid from the space, and having a distal end from which to apply the liquids; and

a hydrophobic film surrounding substantially the entire circumferential surface of the liquid-applying member, contacting the inner surface of the main body, and secured to the main body so as to move, together with the liquid-applying member, in an axial direction of the main body.

The liquid applicator of either type according to the invention comprises a container, a liquid-applying member located in the container, and a hydrophobic film surrounding the liquid-applying member. Hence, when the container is opened, the film prevents the liquid from scattering around to wet the fingers of the person who hold the applicator or the clothes that person wears. The liquid applicator of the invention is, therefore, hygienic. It requires simple manipulation to apply liquid.

This invention can be more fully understood from the following detailed description when taken in conjunction with the accompanying drawings, in which:

Fig. 1 is a partially sectional side view of a liquid applicator according to the present invention;

Fig. 2 is a partially sectional side view of the liquid applicator which has been opened;

Fig. 3 is a cross sectional view, taken along line III-III in Fig. 2; and

Fig. 4 is a partially sectional side view of the liquid applicator which is being used.

A liquid applicator according to an embodiment of the present invention will now be described in detail, with reference to the accompanying drawings.

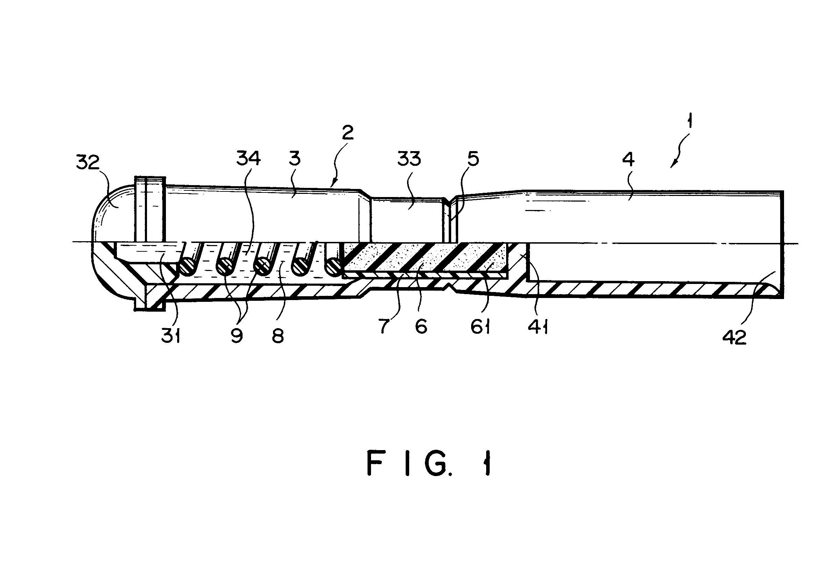

As is shown in Fig. 1, the liquid applicator 1 of the invention comprises a cylindrical container 2 having a closed bottom at one end, and a liquid-applying member 6. The container 2 is a hollow cylinder and comprises a main body 3 and a cap 4. The main body 3 supports the liquid-applying member 6. The cap 4 surrounds at least one portion of the liquid-applying member 6. The main body 3 and the cap 4 are connected together by a thin-walled portion 5 which can be broken by hands.

The liquid applicator 1 further comprises a bottom cap 32. The bottom cap 32 is partially fitted in the open (or proximal) end 31 of the main body 3 (i.e., the left end in Fig. 1), thus closing the open end 31 thereof in airtight fashion. The distal end portion (i.e., the right end portion) of the main body 3 is a supporting portion 33 which holds the liquid-applying member 7 and also a film 7 (later described).

Like the main body 3, the cap 4 is a hollow cylinder. It has a partition 41 in its proximal portion. As long as the cap 4 is connected to the main body 3, more precisely to the supporting portion 33, as is shown in Fig. 1, the cap 4 covers the exposed, distal end 61 of the liquid-applying member 6.

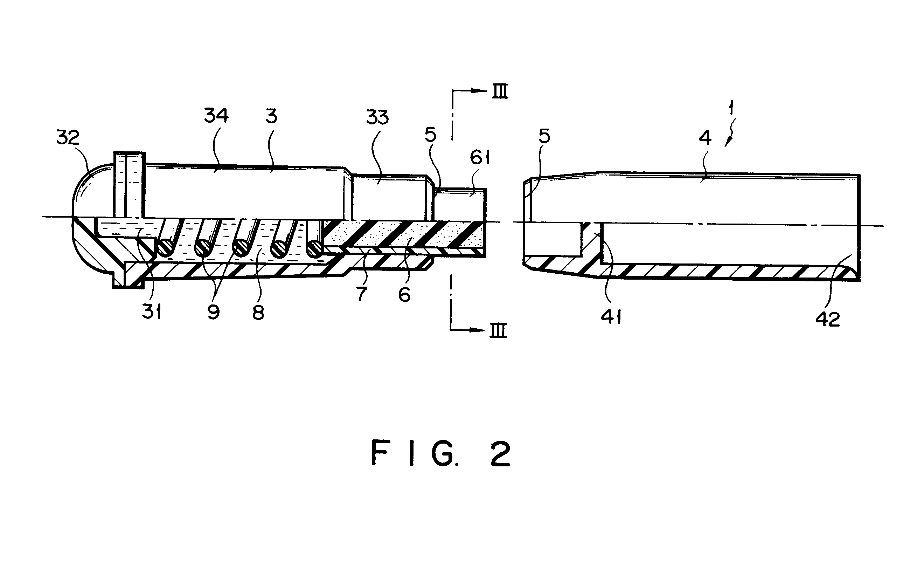

The thin-walled portion 5 is located between the distal end of the main body 3 and the proximal end of the cap 4. The portion 5 is positioned to surround the liquid-applying member 6. The thin-walled portion 5 is formed by cutting an annular V-groove in the outer circumferential surface of the container 2. To use the liquid applicator 1, the container 2 is broken at the thin-walled portion 5 which works as connecting means, thereby separating the main body 3 and the cap 4 from each other, as is illustrated in Fig. 2.

The liquid-applying member 6, which impregnated with liquid 8, is held within the container 2. The member 6 is inactive to the liquid 8 it contains. Preferably, it is made of porous material, either fabric or non-fabric. Non-fabric porous materials which can be used for the member 6 include paper, foamed material, sintered resins, and the like. Fabric porous materials which can be used for the member 6 include woven material, unwoven fabric, knitted material, short-fiber material, long-fiber material, and the like.

Fabric forming the liquid-applying member 6 is selected from natural fibers such as cellulose fiber, cotton, silk and wool, or from resin fibers such as glass fiber, nylon, polypropylene, polyethylene and polyester. Alternatively, the member 6 can be made of any combination of these natural fibers and these resin fibers.

The liquid-applying member 6 is formed either by cutting a block of porous material into a column, or by bending, winding, laminating, or bundling sheets of the porous material to be a columnar member.

The liquid-applying member 6 can be processed and made hydrophilic, so that it may contain more liquid. The member 6 can be made hydrophilic by being immersed in, for example, nonionic surface-active agent (e.g., fatty-acid polyvalent alcohol ester) or anionic surface-active agent and then by being dried.

The columnar liquid-applying member 6 has a diameter of about 1 to 50 mm, preferably 5 to 25 mm, and a length of 5 to 30 mm, preferably 10 to 20 mm. The member 6 remains impregnated with liquid 8, both before the container 2 is open and during the applicator 1 is being used. (The liquid 8 will be described later in detail.)

The soft hydrophobic film 7 is provided around the liquid-applying member 6, covering the entire circumferential surface thereof. Since the film 7 covers the circumferential surface of the member 6, it prevents the liquid 8 from scattering around when the container 2 is broken at the thin-walled portion 5, applying shock to the liquid-applying member 6.

In the container where the member 6 is a bundle of elements, the film 7 binds the elements together, forming a column, and prevents the member 6 from deforming, inflating or disintegrating while the liquid applicator 1 is being used to apply the liquid 8 from the member 6.

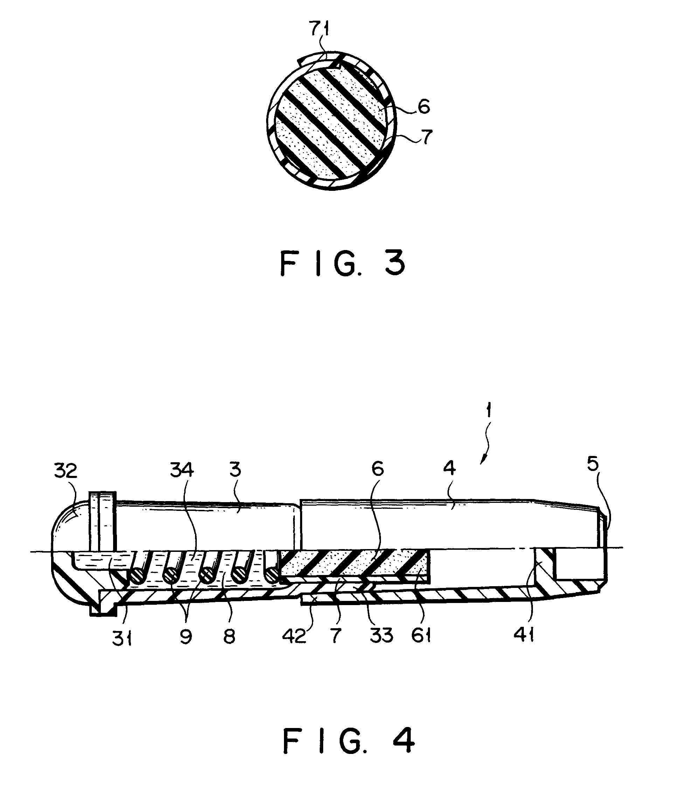

The film 7 should better be made of material which is inactive to the liquid 8 contained in the liquid-applying member 6. More specifically, it can be made of various resins, grease-proof paper, processed paper (e.g., resin-coated paper), synthetic paper, and the like. Of these materials, the most desirable is a resin which can be fusion-welded. This is because a film 7 of the resin, wound around the member 6 as is shown in Fig. 3, can have its overlapping ends 71 fusion-welded together by means of heating, high-frequency, or ultrasonic. Namely, agents (e.g., a solvent or an adhesive) which may adversely influence the liquid 8 are not used to bond together the ends of the resin strip. The resin can be selected from polyethylene, soft polyvinyl chloride, polypropylene, olefin-series copolymer, ionomer resin, thermoplastic elastomer.

The film 7 can be formed of a single layer made of one of the resins mentioned above. Alternatively, it can be formed of two or more layers made of different resins.

It is desirable that overlapping ends 71 of the film 7 be 0.1 to 10 mm long, more preferably about 0.5 to 2 mm long. The film 7 should be 20 to 100 $\mu$m thick, more preferably 20 to 30 $\mu$m thick. If its thickness is less than 20 $\mu$m, it will be deformed as it is cooled and shrinks. If its thickness is greater than 100 $\mu$m, it will become so hard as to give a patient uncomfortable feeling when it touches the affected portion of the patient.

It is preferable that the film 7 covers the entire circumferential surface of the liquid-applying member 6, as is shown in Fig. 1. Nonetheless, the film 7 may not be formed near an end portion (e.g., proximal end portion) of the liquid-applying member 6. Further, the film 7 can be formed by wrapping the liquid-applying member 6 with a strip of the resin, more than once, for example, twice or thrice.

The liquid-applying member 6, thus wrapped with the film 7, is fitted in the supporting portion 33 (i.e., the distal end portion the main body 3), so that it can move in the axial direction of the container 2. The shorter the gap between the film 7 and the inner circumferential surface of the supporting portion 33, the better. The lower the sliding friction between the film 7 and that surface of the supporting portion 33, the better. Therefore, it is desirable that the cylinder of the film 7 have an outside diameter which is nearly equal to the inside diameter of the supporting portion 33.

The space 34 within the main body 3 is filled with liquid 8. The liquid 8 wets the proximal end of the liquid-applying member 6. Hence, as the liquid 8 is applied from the member 6 or evaporates therefrom, the liquid 8 is supplied from the space 34 to the member 6, thus replenishing the member 6 with the liquid 8.

The liquid 8 can be various types, either medical or non-medical. Among medical liquids which may be filled in the space 34 are: liquid medicine, washing liquid, and antiseptic solution. Among non-medical liquids which may be filled in the space 34 are: cosmetics, such as manicure, polish remover, skin cream, lotion, eyeliner, liquid powder, perfume, milky lotion, hair lotion, and hair dye; liquids for use in offices, such as ink, India ink, glue, correction liquid, water colors, oils and solvent; cooking oil and liquid; liquid for industrial use, such as lubricant, anticorrosive, wax, detergent, paint and stain removal.

The liquid 8 can be filled in the main body 3 in any amount desired. Preferably, it is filled in an amount ranging from 0.1 to 5 ml, more preferably from 0.3 to 0.8 ml.

As is evident from in Fig. 1, it is desirable that the liquid-applying member 6, with the film 7 wound around it, be biased toward the cap 4, or to the right in Fig. 1, by a resilient member. In this regard, a coil spring 9 is connected at one end to the inner surface of the cover 32 and at the other end to the proximal end of the member 6. The coil spring 9 can be replaced by a leaf spring or any other resilient member.

Until the container 2 is broken at the think-walled portion 5, the coil spring 9 keeps pushing the liquid-applying member 6 covered with the film 7, onto the partition 41 which is formed in, and integral with, the container 2. When the container 2 is broken at the thin-walled portion 5, whereby the main body 3 and the cap 4 are separated from each other, the distal end portion 61 of the liquid-applying member 6 is exposed. Thereafter, when the member 6 is placed in contact with the object to which to apply the liquid 8.

When the member 6 is pushed into the main body 3, compressing the coil spring 9, the pressure on the liquid 8 filled in the space 34 increases. As a result, the liquid 8 is supplied into the liquid-applying member 6. When the member 6 is released from the pushing force, the coil spring 9 expands to its original length, thus moving the member 6 to the initial position.

The container 2 and the bottom cap 32 should better be made of material which is inactive to the liquid 8 and which can form a gas barrier for preventing, to some extent, the liquid 8 from evaporating. They can be made of various resins including polypropylene, polyethylene, polyvinyl chloride, polyvinylidene chloride, polyethylene telephthalate, polystyrene, polycarbonate, acrylic resin, ABS resin, silicone resin. Alternatively, they can be made of various ceramics such as glass and alumina, or various metals such as stainless steel and aluminum. Of these materials, the most preferable are polypropylene, polyethylene, polyvinyl chloride, and polystyrene. This is because, the container 2, if made of one of these resins, can easily be broken at the thin-walled portion 5, and can easily be manufactured and, thus, at low cost.

The main body 3 and the cap 4 can be separately prepared and then connected together by fusing or bonding, thereby forming the integral container 2. To reduce the number of components of the applicator 1, however, it is more recommendable that the main body 3 and the cap 4 be formed integral of the same material or formed of different colored materials by multi-colored, such as two colored, formation.

The coil spring 8 should also better be made of material which is inactive to the liquid 8. To be more specific, it is desirable that the spring 9 be made of various resins such as polypropylene, polyethylene, polyvinyl chloride, polyethylene telephthalate, polystyrene, polycarbonate, acrylic resin, ABS resin and silicone, or various metals such as stainless steel, aluminum and titanium.

In the embodiment shown in Fig. 1, the coil spring 9 is not integral with the bottom cap 32. However, it may be formed to be integral with the bottom cap 32, thereby to reduce the number of components constituting the applicator 1. In this case, the spring 9 and the bottom cap 32 can be made of either the same material or differently colored materials, such as two colored materials.

Preferably, the liquid applicator 1 is a disposable one, which is thrown away after being used once or a few times. If the applicator 1 is of the type to be thrown away after being used a few times, the liquid-applying member 6 should not be left exposed between uses, so that it may not contaminated or may not let the liquid evaporate. This is why the distal end portion 42 of the cap 4 is so shaped and sized as to hold, preferably in airtight fashion, the distal end of the main body 3 as is shown in Fig. 4.

The present invention is not limited to the liquid applicator shown in Figs. 1 to 4. Rather, various changes and modifications can be made. For instance, the container 2 can be made of a main body and a cap fitted at one end in the main body or connected thereto in screw engagement. Further, the coil spring 9 can be omitted, and the liquid-applying member 6 is secured to the supporting member 33.

The sectional shape of the container of the liquid application according to the invention is not limited to round shapes but can be one of other shapes, such as elliplical shapes and polygonal shapes.

The use of the liquid applicator according to the invention is not necessarily limited. The applicator can find uses in medical treatment, in offices, in food-processing, in cooking, and in manufacturing products.

**Claims**

1. A liquid applicator comprising:
   a container (2) comprising a main body (3), a detachable portion (4), and a connecting means (5) connecting said container (2) and said main body (3) together;
   space-defining means defining a space in said main body;
   an amount of liquid (8) contained in said space;
   a columnar liquid-applying member (6) made of liquid-holding material, located within said container (2) and secured to said main body (3), supplied with the liquid (8) from the space, and having a distal end from which to apply the liquid (8); and
   a hydrophobic film (7) surrounding a circumferential surface of at least that portion of said liquid-applying member (6) which protrudes from said main body (3).

2. The liquid applicator according to claim 1, characterized in that said film (7) is formed on the substantially entire circumferential surface of said liquid-applying member (6) and contacts the inner surface of said main body (3), and said liquid-applying member (6) and said film (7) are secured to said main body (3).

3. The liquid applicator according to claim 2, characterized in that said liquid-applying member (6) and said film (7) are able to move together in an axial direction of said main body (3).

4. The liquid applicator according to claim 3, characterized in that said liquid-applying member (6) and said film (7) extend within said detachable portion (4) of said container.

5. The liquid applicator according to claim 1, characterized in that said film (7) is made of resin which can be fusion-welded.

6. The liquid applicator according to claim 1, characterized in that said connecting means (5) has a thin-walled portion, and said main body (3) and said detachable portion (4) are separated from each other when said connecting means (5) is broken at the thin-walled portion.

7. The liquid applicator according to claim 6, characterized in that said detachable portion (4) has a hollow distal end, which serves as cap means for fitting on said main body (3) so as to house said liquid-applying member (6) after said main body (3) has been separated from said detachable portion (4).

8. The liquid application according to claim 3, characterized in that said liquid-applying member (6) is connected to said main body (3) by a coil spring (9) arranged in said space.

9. A liquid applicator comprising:
   a container (2) comprising a main body (3), a detachable portion (4), and a connecting means (5) connecting said container (2) and said main body (3) together;
   space-defining means defining a space in said main body;
   an amount of liquid (8) contained in said space;
   a columnar liquid-applying member (6) made of liquid-holding material, located within said container (2) and extending between said main body (3) and said detachable portion (4), supplied with the liquid (8) from the space, and having a distal end from which to apply the liquid (8); and
   a hydrophobic film (7) surrounding substantially the entire circumferential surface of said liquid-applying member (6), contacting the inner surface of said main body (3), and secured to said main body (3) so as to move, together with said liquid-applying member (6), in an axial direction of said main body (3).

10. The liquid applicator according to claim 9, characterized in that said film (7) is made of resin which can be fusion-welded.

11. The liquid applicator according to claim 10, characterized in that said connecting means (5) has a thin-walled portion, and said main body (3) and said detachable portion (4) are separated from each other when said connecting means (5) is broken at the thin-walled portion.

12. The liquid applicator according to claim 11, characterized in that said detachable portion (4) has a hollow distal end, which serves as cap means for fitting on said main body (3) so as to house said liquid-appliying member (6) after said main body (3) has been separated from said detachable portion (4).

13. The liquid application according to claim 9, characterized in that said liquid-applying member (6) is connected to said main body (3) by a coil spring (9) arranged in said space.

F I G. 1

EP 0 475 425 A1

F I G. 2

F I G. 3

F I G. 4

European
Patent Office

**EUROPEAN SEARCH REPORT**

Application Number

**EP 91 11 5538**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | WO-A-8 905 695 (TERUMO KABUSHIKI KAISHA)<br>* abstract; figures 1A-1C *<br>– – – – – | 1,3,4,6-9,<br>11-13 | A 61 M 35/00<br>A 45 D 34/04<br>B 05 C 17/00 |

| | |
|---|---|
| | TECHNICAL FIELDS SEARCHED (Int. Cl.5) |
| | A 45 D<br>B 05 C<br>A 61 M |

The present search report has been drawn up for all claims

| Place of search | Date of completion of search | Examiner |
|---|---|---|
| Berlin | 13 December 91 | ROLAND A T |